(19) Europäisches Patentamt
European Patent Office
Office européen des brevets

(11) EP 4 219 596 A1

(12) **EUROPEAN PATENT APPLICATION**
published in accordance with Art. 153(4) EPC

(43) Date of publication:
**02.08.2023 Bulletin 2023/31**

(21) Application number: **21872645.3**

(22) Date of filing: **06.10.2021**

(51) International Patent Classification (IPC):
***C08G 77/38*** (2006.01) ***C08G 77/50*** (2006.01)
***C08L 83/10*** (2006.01) ***C08L 83/14*** (2006.01)
***A61K 8/892*** (2006.01) ***C08K 3/00*** (2018.01)

(52) Cooperative Patent Classification (CPC):
**A61K 8/04; A61K 8/28; A61K 8/29; A61K 8/892; A61K 8/894; A61Q 1/02; C08G 77/38; C08G 77/50; C08K 3/00; C08L 83/10; C08L 83/14**

(86) International application number:
**PCT/JP2021/037003**

(87) International publication number:
**WO 2022/065520 (31.03.2022 Gazette 2022/13)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **25.09.2020 JP 2020160317**

(71) Applicant: **Dow Toray Co., Ltd.**
**Tokyo 140-8617 (JP)**

(72) Inventors:
- **KIKUNAGA Sayuri**
  **Ichihara-shi Chiba 299-0108 (JP)**
- **HORI Seiji**
  **Ichihara-shi Chiba 299-0108 (JP)**
- **KANZAKI Yasue**
  **Ichihara-shi Chiba 299-0108 (JP)**

(74) Representative: **Murgitroyd & Company**
**Murgitroyd House**
**165-169 Scotland Street**
**Glasgow G5 8PL (GB)**

# (54) CO-MODIFIED ORGANOPOLYSILOXANE AND USE THEREOF

(57) [Problem]

An object of the present invention is to provide: a co-modified organopolysiloxane that has excellent affinity and compatibility with a wide range of cosmetic product raw materials (particularly non-silicone oil agents such as fatty acid ester oils and the like), is less likely to cause thickening or inferior dispersion when used with powders, and can form favorable and stable slurries; and a use thereof.

[Resolution Means]

A co-modified organopolysiloxane expressed by the following structural formula (1), with an HLB value within a range of 0.5 to 2.0 and viscosity within a range of 200 to 1500 mPa s, as well as a use thereof as a powder dispersing agent or the like. $R^2$ is a monovalent hydrocarbon group having 6 to 30 carbon atoms, $L^1$ is an organosilicon-containing organic group selected from silylalkyl groups having a carbosiloxy dendron structure and groups having a siloxane macromonomer structure, and Q is a hydrophilic group, which is a (poly)glycerin residual group or sugar alcohol residual group}.

EP 4 219 596 A1

## Description

## TECHNICAL FIELD

The present invention relates to: a co-modified organopolysiloxane having a specific silicon-containing organic group, hydrophilic group, and specific hydrocarbon group such as a long chain alkyl group or the like in specific ratios in the molecule, and having a certain HLB value and viscosity, thus having excellent affinity with a wide variety of oil agents and being highly useful as a powder treatment agent. The present invention also relates to a use thereof.

## BACKGROUND ART

In topical agent compositions including cosmetic products, various types of powders represented by titanium dioxide and other white and colored pigments, mica and other extender pigments, and powder dispersions thereof in oil agents are widely used as cosmetic raw material and the like. In order to improve the dispersion properties, compounding stability, and feel/function of the cosmetic materials containing powders and the like, powders treated with a surface treatment by various siloxane components are used (for example, Patent Documents 1 to 6). However, the performance of conventional powder treatment siloxanes is insufficient, and the present applicant has proposed a co-modified organopolysiloxane copolymer having a group with a carbosiloxy dendron structure or the like, a glycerin derivative or other hydrophilic group, and an alkyl group in a molecule, and having low molecular weight and low viscosity, and has also proposed use of this copolymer as a powder treatment agent (Patent Document 7).

On the other hand, the co-modified organopolysiloxane specifically disclosed in Patent Document 7 achieves a higher powder dispersion performance than conventional siloxane components, but when a powder dispersion is formed with an oil agent in some low HLB regions, thickening, reduced fluidity, and the like may occur over time, leaving room for improvement in the stability over time. In addition, in recent years, from the perspective of further improving the degree of freedom in formulation design of cosmetic materials and the like, there is demand in the market for a co-modified organopolysiloxane that has a high affinity for a wide variety of oil agents and that can form a powder dispersion with excellent stability over time.

## RELATED ART DOCUMENTS

## PATENT DOCUMENTS

Patent Document 1: Japanese Unexamined Patent Application H07-53326 (Japanese Patent No. 2719303)
Patent Document 2: Japanese Unexamined Patent Application H10-167946
Patent Document 3: Japanese Unexamined Patent Application 2002-38013
Patent Document 4: International Published Patent WO2011/049246
Patent Document 5: International Published Patent WO2011/049248
Patent Document 6: International Published Patent WO2011/136394
Patent Document 7: Japanese Unexamined Patent Application 2013-151657 (Japanese Patent No. 6369887)

## SUMMARY OF THE INVENTION

## PROBLEM TO BE SOLVED BY THE INVENTION

In light of the foregoing, an object of the present invention is to provide a co-modified organopolysiloxane that has excellent affinity and compatibility with a wide range of cosmetic product raw materials (in particular, different types of oil agents), is less likely to cause thickening or inferior dispersion when used with powders, and can form favorable and stable slurries. Furthermore, another object of the present invention is to provide: a powder treatment agent containing the organopolysiloxane; a powder surface treated by the powder treatment agent; a powder composition containing the co-modified organopolysiloxane copolymer; a powder-in-oil dispersion containing an oil agent; a topical agent composition containing these, and particularly a make-up cosmetic material; and a manufacturing method thereof.

## MEANS FOR SOLVING THE PROBLEM

As a result of careful examination, the present inventors discovered that the problems above can be solved by a straight-chain co-modified organopolysiloxane with a relatively low degree of polymerization, having an HLB within a

range of 0.5 to 2.0, having a viscosity at 25°C within a range of 200 to 1500 mPa s, and having in a molecule a specific hydrophilic group (Q), a C6 to C30 long chain hydrocarbon group (R2), and an organosilicon-containing organic group (L1) selected from silylalkyl groups having a carbosiloxy dendron structure and groups having a siloxane macromonomer structure in a specific ratio in a side chain portion, thereby arriving at the present invention. Furthermore, the present inventors discovered that the above problems can be solved by a powder treatment agent containing the co-modified organopolysiloxane, a powder composition, a powder-in-oil dispersion containing an oil agent, a topical agent composition containing these, and particularly a make-up cosmetic material, as well as a manufacturing method thereof, thereby arriving at the present invention. Note that the co-modified organopolysiloxane according to the present invention has excellent affinity and compatibility not only with silicone oils but also with non-silicone organic oil agents (e.g., hydrocarbon oils and fatty acid ester oils), making them particularly suitable for use in cosmetic materials and other topical agent compositions that combine different types of oil agents and contain powder.

## EFFECTS OF THE INVENTION

The present invention can provide a co-modified organopolysiloxane that has excellent affinity and compatibility with a wide range of cosmetic product raw materials (in particular, different types of oil agents), is less likely to cause thickening or inferior dispersion when used with powders, and can form a favorable and stable slurry. Furthermore, the present invention provides: a powder treatment agent containing the organopolysiloxane; a powder surface treated by the powder treatment agent; a powder composition containing the co-modified organopolysiloxane copolymer; a powder-in-oil dispersion containing an oil agent; a topical agent composition containing the powder-in-oil dispersion, and particularly a make-up cosmetic material; and a manufacturing method thereof.

## DESCRIPTION OF THE PREFERRED EMBODIMENT

Hereinafter, the co-modified organopolysiloxane of the present invention, use thereof in various types of treatment agents (surfactants or surface treatment agents), and in particular, use as a powder treatment agent, and use as a cosmetic raw material will be described below in detail. Furthermore, detailed descriptions will be provided of a powder-in-oil dispersion, a topical agent, preferably a cosmetic material, and particularly preferably a make-up cosmetic material using the co-modified organopolysiloxane of the present invention. Note that the co-modified organopolysiloxane of the present invention can be applied to applications in common with the co-modified organopolysiloxanes described in Patent Document 5 (International Published Patent WO2011/049248) and Patent Document 7 (Japanese Unexamined Patent Application 2013-151657) described above, and can be used to replace some or all of the co-modified organopolysiloxanes disclosed in these patent documents. Furthermore, for the dosage forms, types, and formulation examples of cosmetic materials and the like disclosed in these patent documents, it is possible to provide a cosmetic material, powder dispersion, emulsion, and the like by replacing some or all of the co-modified organopolysiloxanes used therein with the co-modified organopolysiloxane according to the present invention, and the present invention strongly teaches such formulation design.

The co-modified organopolysiloxane according to the present invention is a chain polysiloxane having a trimethylsiloxy end, expressed by structural formula (1) described later, where a side chain portion has an organosilicon-containing organic group (L1) selected from silylalkyl groups having a carbosiloxy dendron structure and groups having a siloxane macromonomer structure, a specific hydrophilic group (Q), and a long chain hydrocarbon group (R2) having 6 to 30 carbon atoms at a specific ratio; therefore, the HLB is in a range of 0.5 to 2.0, and the viscosity at 25°C is in a range of 200 to 1500 mPa s.

In the present invention, the hydrophilic-lipophilic balance (HLB) value is defined by "(total molecular weight of hydrophilic group portion/total molecular weight) $\times$ 20". For the co-modified organopolysiloxane of the present invention, the value must be in a range of 0.5 to 2.0, and from the perspective of performance as a powder dispersing agent, the value may be in a range of 1.1 to 2.0, and is particularly preferably in a range of 1.5 to 2.0. In structural formula (1) described below, the values of a to d, and particularly the value of c, which affects the number of bonds of the hydrophilic group (Q), must be values that satisfy the aforementioned range of HLB values for the entire molecule.

If the HLB value is outside the range above, and particularly if the HLB value is less than the lower limit and the amount of hydrophilic group (Q) in the molecule is low, even the co-modified organopolysiloxane expressed by structural formula (1) may not be able to fully coat the solid surface, and a slurry thereof may have a tendency to thicken over time, resulting in insufficient performance as a powder treatment agent. On the other hand, when the HLB value is greater than 2.0, that is, when the amount of hydrophilic groups in the molecule increases, the hydrophilic group (Q) according to the present invention, and preferably a diglycerin derivative group-containing an organic group, sugar alcohol-modified group, or the like, has many hydroxyl groups. Therefore, the viscosity of the co-modified organopolysiloxane itself tends to increase due to the cohesive force caused by hydrogen bonding, which may result in insufficient handling workability when used as a powder treatment agent.

As described above, from the perspective of handling workability as a powder treatment agent, the co-modified organopolysiloxane according to the present invention must have a viscosity in a range of 200 to 1500 mPa·s at 25°C. In particular, from the perspective of affinity with respect to a non-silicone organic oil agent (e.g., hydrocarbon oils and fatty acid ester oils), the viscosity is preferably within a range of 300 to 1450 mPa s, and more preferably 700 to 1450 mPa s. Even if the co-modified organopolysiloxane has the HLB described above and has a specific hydrophilic group, an organosilicon-containing organic group, and a monovalent hydrocarbon group having 6 to 30 carbon atoms, if the viscosity thereof exceeds the aforementioned upper limit, the handling workability as a powder treatment agent will be reduced, and in particular, the work efficiency and dispersion when preparing the powder dispersion will be insufficient when using a non-silicone organic oil agent (e.g. hydrocarbon oil and fatty acid ester oil). Furthermore, the resulting powder dispersion (in particular, a slurry, which is a powder-in-oil dispersion) may thicken over time, or the dispersion properties of the powder may be insufficient, making it difficult to use as a cosmetic raw material. Moreover, depending on the conditions, it may not be possible to prepare a powder dispersion at all.

Note that the viscosity of the co-modified organopolysiloxane is greatly affected by the type and amount of the hydrophilic group (Q) as well as the degree of polymerization n1 to n4 of each diorganosiloxy unit in general formula (1) and the sum (n1+n2+n3+n4). Therefore, the values of n1 to n4 in structural formula (1) must be within the viscosity range described above, and it is particularly preferable that the range of (n1 + n2 + n3 + n4) is 8 to 25, n1 is 3 to 10, n2 is 1 to 10, n3 is 0.1 to 10, and n4 is 0.5 to 2.0.

The co-modified organopolysiloxane according to the present invention is expressed by the following structural formula (1):

[Formula 1]

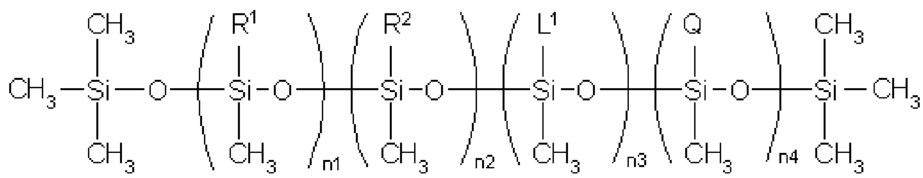

(1)

In the formula, $R^1$ is a substituted or unsubstituted monovalent hydrocarbon group having 1 to 30 carbon atoms or is a hydrogen atom, not including other groups corresponding to $R^2$, L', and Q, but a methyl or phenyl group is industrially preferable.

$R^2$ is a substituted or unsubstituted, straight-chain or branched monovalent hydrocarbon group having 6 to 30 carbon atoms, and can particularly improve affinity with non-silicone oil agents such as hydrocarbon-based oil agents (cosmetic raw materials). Suitably, $R^2$ is an alkyl group having 6 to 30 carbon atoms, and examples include hexyl groups, heptyl groups, octyl groups, decyl groups, dodecyl groups, undecyl groups, and hexadecyl groups, but alkyl groups having 8 to 20 carbon atoms are particularly preferred.

$L^1$ is an organosilicon-containing organic group selected from silylalkyl groups having a carbosiloxy dendron structure and groups having a siloxane macromonomer structure. Specifically, if i = 1, $L^1$ in the present invention is a one or more type selected from:

a silylalkyl group having a siloxane dendron structure, expressed by the following general formula (2):

[Formula 2]

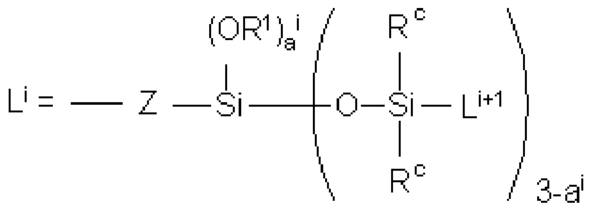

(2)

(where $R^1$ is a group similar to the groups described above, $R^C$ is an alkyl group with 1 to 6 carbon atoms, or a phenyl group, and Z is a divalent organic group.
i indicates the number of units of silylalkyl groups represented by L' and is an integer from 1 to c where c represents the number of units, or in other words the number of repetitions of the silylalkyl group, and the number of units c is an integer from 1 to 10. $L^{i+1}$ represents a silylalkyl group when i is less than c, and is a methyl group or a phenyl group when i=c.
$a^i$ is a number from 0 to 3);
a chain organosiloxane group, expressed by the following general formula (2'):

[Formula 3]

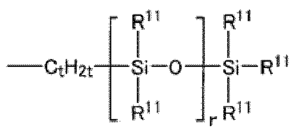

(2')

(where $R^{11}$ are each independently a substituted or unsubstituted monovalent hydrocarbon group having 1 to 30 carbon atoms, hydroxyl group, or hydrogen atom, and at least one of the $R^{11}$ groups is a monovalent hydrocarbon group. t is a number in a range of 2 to 10; and r is a number in a range of 1 to 500); and
a chain type organosiloxane group expressed by the following general formula (2"):

[Formula 4]

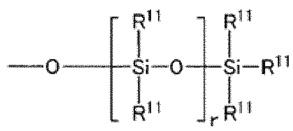

(2")

(where $R^{11}$ and r are similar to those described above).

The groups represented by $L^1$ may be the same or different. For example, a silylalkyl group having a carbosiloxy dendron structure and a group having a siloxane macromonomer structure may be present in the same molecule. The silylalkyl group may be a combination of organosilicon-containing organic groups with different degrees of branching

(generations), and the group having a siloxane macromonomer structure may be a combination of organosilicon-containing organic groups with different degrees of polymerization.

The group expressed by $L^1$ may be a silylalkyl group having a carbosiloxane dendrimer structure and is defined as the silylalkyl group expressed by the above general formula (2) when i = 1. The silylalkyl group having the carbosiloxane dendrimer structure has a structure in which the carbosiloxane units are extended in the form of a dendrimer. Therefore, the silylalkyl group is a functional group exhibiting increased water repellency in comparison to straight-chain or simply branched polysiloxane units, and can provide an excellent surfactant and powder treatment agent that are excellent in the co-modified organopolysiloxane according to the present application, without impeding the feel derived from the hydrophilic functional group. Furthermore, the silylalkyl group having a carbosiloxane dendrimer structure is chemically stable, and therefore, the silylalkyl group is a functional group providing advantageous properties that can be used in combination with a wide range of cosmetic material components.

In general formula (2), $R^C$ is an alkyl group with 1 to 6 carbon atoms or a phenyl group, but methyl or phenyl groups are industrially preferable.

In general formula (2), i represents the number of units of silylalkyl groups expressed by $L^1$ and is an integer from 1 to c, where c represents the number of units, or in other words the number of repetitions of silylalkyl groups, and where the number of units c is an integer from 1 to 10. $L^{i+1}$ represents a silylalkyl group when i is less than c, and is a methyl group or a phenyl group when i=c. In particular, it is preferably a methyl group when i = c.

Note that the number of units c is preferably an integer from 1 to 3 and is more preferably 1 or 2 from an industrial standpoint.

For each of the number of units, the group expressed by L1 is expressed as follows. In the formula, R2 and Z are similar to the groups described above.

If the number of units c = 1, $L^1$ is expressed by the following general formula (2-1).

[Formula 5]

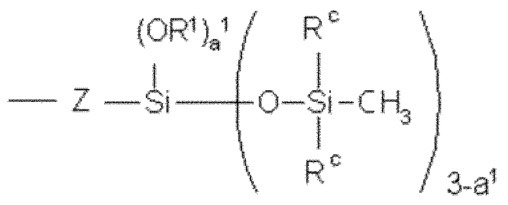

(2-1)

When the number of units is c = 2, $L^1$ is expressed by the following general formula (2-2).

[Formula 6]

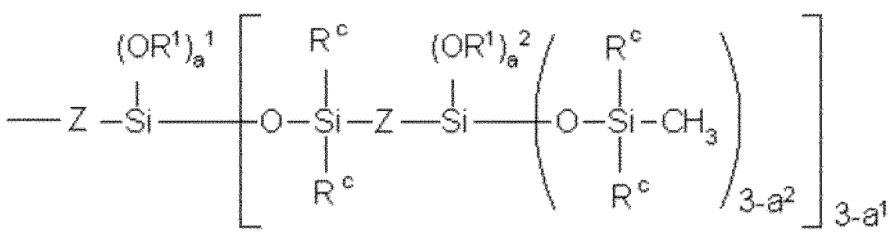

(2-2)

In formula (2), ai independently represents a number in a range of 0 to 3. In the structures expressed in formulas (2-1) to (2-3) where the number of units is 1 to 3, a1, a2, and a3 independently represent a number in a range of 0 to 3. ai values are preferably numbers within a range of 0 to 1, and ai is particularly preferably 0.

In the formula, Z are each independently a divalent organic group, and specific examples thereof include a divalent organic group formed by addition-reacting a silicon-bonded hydrogen atom and a functional group having an

unsaturated hydrocarbon group such as an alkenyl group, an acryloxy group, a methacryloxy group, or the like at an end. Depending on the method for introducing the silylalkyl group having a carbosiloxane dendrimer structure, the functional group can be appropriately selected and is not limited to the functional groups described above. Although a plurality of such functional groups are disclosed in Patent Document 7 and the like, Z in the present invention is preferably an alkylene group with 2 to 10 carbon atoms, but ethylene groups are most preferable.

The group expressed by $L^1$ may be a siloxane macromonomer structure-containing group bonded to silicon atoms configuring the main chain via an oxygen atom or an alkylene group, and is a functional group that is hydrophobic and exhibits certain water-repellent/lipophilic properties, and provides a surfactant and powder treatment agent that are excellent in the organopolysiloxane copolymer according to the present application, without impairing the feel derived from hydrophilic functional groups. Specifically, the group expressed by $L^1$ may be a functional group expressed by the general formula (2') and general formula (2"). In the formula, $R^{11}$ is particularly industrially preferably a methyl group, phenyl group or hydroxyl group, and at least a portion of $R^{11}$ is a methyl group, and a portion is preferably in the form of a long chain alkyl group with 8 to 30 carbon atoms. However, at least one $R^{11}$ is a monovalent hydrocarbon group such as a methyl group of the like, t is a number in a range of 2 to 10, and r is a number in a range of 1 to 500. From the perspective of compatibility with various oil agents, r is preferably a number in a range of 1 to 100, and r is particularly preferably a number in a range of 2 to 30.

Q is a hydrophilic group containing a sugar alcohol expressed by the following structural formulae (3-1) to (3-2):

[Formula 7]

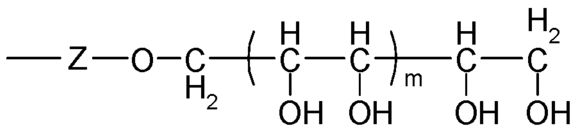

(3-1)

(where Z represents an alkylene group having 2 to 22 carbon atoms or a divalent organic group expressed by $-R^6-C(=O)-O-R^7-$ ($R^6$ is an alkylene group having 2 to 22 carbon atoms, $R^7$ is a group selected from ethylene groups, propylene groups, methylethylene groups, and hexylene groups), and m is 1 or 2)

[Formula 8]

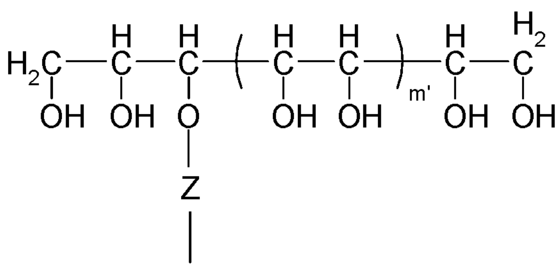

(3-2)

(where Z is similar to those described above, and m' is 0 or 1), and

a hydrophilic group containing at least one hydrophilic unit selected from hydrophilic units expressed by the following structural formulae (3-3) to (3-5):

[Formula 9]

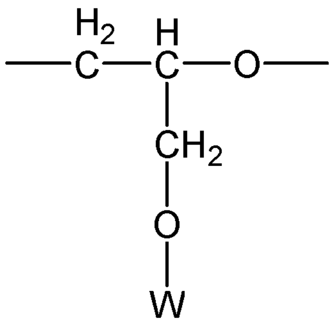

(3-3)

(where W is a hydrogen atom or an alkyl group having 1 to 20 carbon atoms)

[Formula 10]

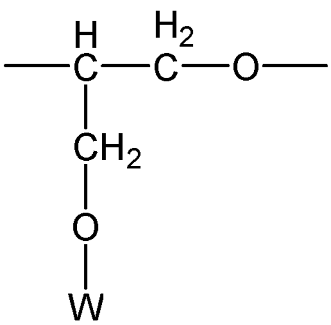

(3-4)

[Formula 11]

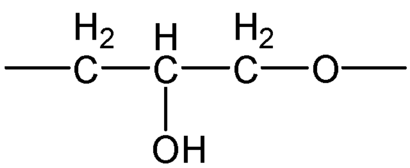

(3-5)

where the hydrophilic groups are being bonded to silicon atom via a divalent linking group (Z).

The hydrophilic group Q is a portion that imparts hydrophilicity to the co-modified organopolysiloxane according to the present application and is generally a functional group derived from a hydrophilic compound. Preferred examples of Q defined as describe above include functional groups derived from monohydric or higher alcohols, polyether-based compounds, (poly)glycerin-based compounds, (poly)glycidyl ether-based compounds, and hydrophilic sugars, where the ends of the molecular chain may be partially blocked with hydrocarbons.

For the co-modified organopolysiloxane according to the present invention, from the perspective of properties as a powder treatment agent, and particularly dispersion properties of inorganic powders, Q is preferably a polyhydric alcohol-containing organic group in the structural formula (1) and the like above, and Q is particularly preferably a (poly)glycerin residual group or a sugar alcohol residue. From the perspective of performance as a powder treatment

agent, and particularly from the perspective of controlling the overall viscosity of the co-modified organopolysiloxane, Q is a hydrophilic group derived from a (poly)glycerol compound, and is most preferably a hydrophilic group derived from (poly)glycerin. Specifically, a preferred hydrophilic group Q is a (poly)glycerinmonoallyl ether or a (poly)glyceryl eugenol, which are derived from (poly)glycerin compounds having a monoglycerin, diglycerin, triglycerin, or tetraglycerin structure, with a hydrophilic group of a diglycerin derivative being optimal.

The diglycerin derivative group is more preferably a diglycerin derivative group expressed by the following structural formula (5):

$$-R^5-O-X_m-H \qquad (5)$$

In the formula, $R^5$ is a divalent organic group containing no oxyalkylene structure with an average number of repetitions of oxyalkylene units of 2 or more, and is preferably an alkylene group having 2 to 10 carbon atoms. X is one of the hydrophilic units selected from the hydrophilic units (= glycerin units) expressed by the structural formulae (3-3) to (3-5) above, and m is the number of repetitions of the glycerin units, which is on average in a range of 1.5 to 2.4. Note that the average value of the number of repetitions of glycerin units is in a range of 1.1 to 2.9, preferably in a range of 1.5 to 2.4, more preferably in a range of 1.8 to 2.2, and is most preferably 2 on average. In other words, the glycerin derivative group serving as Q is primarily a diglycerin derivative group. Herein, the repeating structure of the diglycerin derivative group of glycerin units is preferably not branched, but a part of the structure may have a partially branched structure, such as a polyglycerol group or a polyglycidyl ether group.

The co-modified organopolysiloxane is most preferably a diglycerin derivative group-containing organic group expressed by the following general formula (5-1)

[Formula 12]

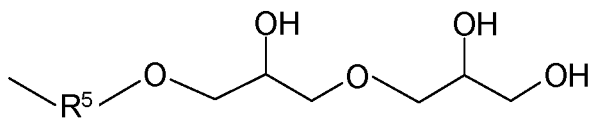

(5-1)

(where $R^5$ represents a divalent organic group not having an oxyalkylene structure having an average number of repetitions of an oxyalkylene unit of 2 or higher),
or
the following general formula (5-2)

[Formula 13]

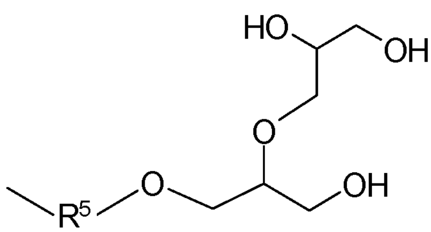

(5-2)

(where $R^5$ is similar to those described above).

In the co-modified organopolysiloxane according to the present invention, the diglycerin derivative group-containing organic group is preferably a hydrophilic group derived from diglycerin monoallyl ether or diglyceryl eugenol. Most preferred is a pure product with a purity of more than 98 mass% of the diglycerin derivative group where m = 2 in

formula (5).

The sugar alcohol-containing organic group serving as Q is particularly preferred when Z is a propylene group and m = 1 in the structural formula (3-1). Similarly, the sugar alcohol-containing organic group is particularly preferred when Z is a propylene group and m' = 0 in the structural formula (3-2). The sugar alcohol-containing organic group in this case is a xylitol residual group expressed by the structural formula:

$$-C_3H_5-OCH_2[CH(OH)]_3CH_2OH$$

or
the structural formula:

$$-C_3H_6-OCH\{CH(OH)CH_2OH\}_2$$

which correspond to the structural formulae (3-1) and (3-2).

In structural formula (1), with n1 to n4 representing the average degree of polymerization of the diorganosiloxy unit, (n1 + n2 + n3 + n4) is a number in a range of 2 to 50, preferably 5 to 40, and particularly preferably 8 to 25. n1 is a number in a range of 0 to 20, and more preferably from 3 to 10. n2 is a number in a range of 1 to 10, and more preferably from 1.0 to 5.0. n3 is a number in a range of 0.1 to 10, and more preferably from 0.1 to 5. n4 is a number in a range of 0.3 to 2.5, and more preferably from 0.5 to 2.0. Furthermore, n4 affects the amount of the hydrophilic group Q. Therefore, n4 is particularly preferably a number in a range where the HLB value of the entire molecule is 0.5 to 2.0, more preferably 1.5 to 2.0.

The co-modified organopolysiloxane according to the present invention can be obtained by addition reacting a compound having an organosilicon-containing organic group having one carbon-carbon double bond at one end of the molecular chain (e.g., the silylalkyl group described above), a hydrophilic compound having a reactive functional group such as an alkenyl group or the like (e.g. diglycerin monoallyl ether or the like), and an unsaturated long chain hydrocarbon compound having a carbon-carbon double bond at one end of the molecular chain, with the organopolysiloxane having a reactive functional group such as Si-H or the like. In the reaction, it is preferable to perform a multi-step addition reaction of the raw materials described above on organopolysiloxanes with reactive functional groups such as Si-H or the like, and from the perspective of reaction control, purity, and yield, it is preferable to perform the addition reaction in the presence of a hydrosilylation reaction catalyst. In addition, a crude product of the co-modified organopolysiloxane obtained by an addition reaction may be purified by performing deodorizing treatment by means of a hydrogenation reaction in a solvent or without a solvent in the presence of a hydrogenation catalyst, or odor reduction treatment may be performed with an acidic substance. Furthermore, the co-modified organopolysiloxane according to the present invention may be obtained as a mixture with a hydrophilic compound having a reactive functional group such as an alkenyl group or the like.

In the synthesis of the co-modified organopolysiloxane according to the present invention, a method common to the reaction, purification, odor reduction treatment with an acidic substance, and the like disclosed by the applicants in paragraphs [0110] to [0122] of Patent Document 5 (International Published Patent WO/2011/049248) can be used. In particular, it is possible to make the co-modified organopolysiloxane substantially odorless. Therefore, treatment is performed with one or more acidic inorganic salts (preferably, hydrogen sulfate sodium or the like) which is solid at 25°C, water soluble and has a pH of 4 or less at 25°C in an aqueous solution of 50 g dissolved in 1 L of ion exchanged water. For example, this refers to: (1) decomposition treatment performed by adding the acidic inorganic salt to a reaction system of a co-modified organopolysiloxane composition synthesized by a hydrosilylation reaction (e.g. in a flask or other reaction container), and then stirring; (2) hydrolysis treatment performed by adding an acidic inorganic salt and water, or an acidic inorganic salt, water, and a hydrophilic solvent and then stirring; and the like. The treatment process that uses the acidic inorganic salt is preferably carried out in the presence of water and/or a hydrophilic solvent.

After the odor reduction treatment described above, it is preferable to include a stripping step for removing low-boiling components (propionaldehyde and the like) that are odor-causing substances, and the treatment with the acidic substance and the stripping of odor-causing substances are preferably performed a plurality of times.

Furthermore, after the acid treatment step, it is particularly preferable from the perspective of odor reduction to add an alkali buffer agent (sodium phosphate, potassium phosphate, sodium citrate, sodium acetate, sodium hydrogen carbonate, or the like) at an amount corresponding to 100 ppm to 50,000 ppm to the resulting co-modified organopolysiloxane or a composition containing the same for neutralization.

From the perspective of removing unwanted residues from the co-modified organopolysiloxane according to the present invention, filtration may be performed by a known method.

Applications of the co-modified organopolysiloxane

The novel co-modified organopolysiloxane according to the present invention (hereinafter, referred to as "component (A)") is hydrophobic, has a silylalkyl group having a siloxane dendron structure that provides high water repellency and a hydrophilic group in the same molecule, and has low viscosity and generally low HLB. Therefore, handling, working efficiency, and compounding stability with lipophilic raw materials are superior. As a result, the novel co-modified organopolysiloxane according to the present invention is useful as various types of treatment agents and cosmetic raw material components, particularly as a surfactant or a surface treatment agent for use in a cosmetic material, and more particularly is extremely useful as a powder treatment agent or powder dispersing agent for use in surface treating a powder or dispersing a powder.

The use of the novel co-modified organopolysiloxane according to the present invention as surfactants is common to the use of the co-modified organopolysiloxane disclosed by the applicants in paragraphs [0124] to [0147] of Patent Document 5 (International Published Patent WO2011/049248) above and the preparation of emulsification compositions. When preparing various emulsification compositions, the co-modified organopolysiloxane (low viscosity, and preferably low HLB) according to the present invention can also be emulsified in combination with the co-modified organopolysiloxane disclosed in Patent Document 5.

[Use as a powder treatment agent or powder dispersing agent]

The co-modified organopolysiloxane according to the present invention has a low viscosity and low HLB and provides an appropriate degree of water repellency due to being oriented on the surface of various powders. Therefore, the co-modified organopolysiloxane according to the present invention can be suitably used as a powder surface treatment agent for the purpose of surface treating or dispersing a cosmetic material powder. In particular, when used as a powder treatment agent, dispersion stability in a mixed oil agent-based, and particularly a non-silicone organic oil agent of the co-modified organopolysiloxane according to the present invention is more favorable compared to known co-modified organopolysiloxanes. Thus, a powder-in-oil dispersion having superior stability in which the powder does not agglomerate or precipitate after preparing a powder composition obtained by treating the powder surface using a treatment agent can be provided, even when a method is used where the powder composition is dispersed in an oil agent dispersing medium and even for powders in which conventional powder treatment agents result in difficulties in stable dispersion.

Other Uses

The novel co-modified organopolysiloxane according to the present invention can also be used as a feel improving agent, a moisturizing agent, a binder, and a skin adhesive/pressure-sensitive adhesive. Additionally, the diglycerin derivative-modified silicone according to the present invention can be combined with water for use as a film agent or a viscosity adjusting agent.

The co-modified organopolysiloxane of the present invention has excellent compatibility with various other hydrophilic and hydrophobic components in the cosmetic composition, and can enhance the dispersibility and stability of a powder in a cosmetic composition that comprises a powder. Thus, the powder treatment agent of the present invention and the powder surface treatment agent of the present invention can improve the stability of a cosmetic composition that comprises a powder and can improve the uniform dispersibility of this powder. A cosmetic composition that comprises a powder that is surface treated using the powder surface treatment agent has high stability and this powder uniformly disperses in this cosmetic composition.

A compounded amount of the co-modified organopolysiloxane in the powder treatment agent of the present invention is not particularly limited provided that powder treatment effects are displayed and, for example, can be from 50 to 100 wt.% (mass%), and is preferably from 70 to 100 wt.%, and more preferably from 90 to 100 wt.%.

When using the co-modified organopolysiloxane according to the present invention as a powder surface treatment agent, the compounded amount of the co-modified organopolysiloxane and the powder or coloring agent is preferably in a range of 0.1 to 30 mass parts, and particularly preferably 0.5 to 10 mass parts with respect to 100 mass parts of the powder or coloring agent. If the compounded amount is less than the lower limit described above, effects by the surface treating may be insufficient. On the other hand, even if the compounded amount exceeds the upper limit described above, greater prominent changes in texture will not occur, and the tendency for the powder and the co-modified organopolysiloxane to form a uniform mixture will increase.

The co-modified organopolysiloxane according to the present invention can be used to treat a powder surface using a conventional method. This method is not particularly limited and, for example, can be appropriately selected from the methods described below.

1. Method of surface treating a target powder by dispersing in a medium selected from organic solvents in which a

treatment agent has been compounded.

2. Method of mixing a powder with a powder treatment agent and then surface treating the mixture using a pulverizer such as a ball mill, a jet mill, or the like.

3. Treatment method of compounding a treatment agent in a solvent, dispersing the powder therein to adsorb to the surface, and then performing drying and sintering.

Powder composition

Additionally, the present invention relates to a powder composition comprising

(A) the co-modified organopolysiloxane according to the present invention and

(B) a powder or coloring agent. The powder composition can be obtained, according to the methods described above or the like, by mixing (B) the powder or coloring agent and (A) the co-modified organopolysiloxane according to the present invention, regardless of the purpose (i.e. to surface treat the powder, improve dispersibility of the powder, to act as a premix for a cosmetic raw material, or the like).

Powder-in-oil dispersion

Additionally, "powder-in-oil dispersion" as used in the present invention, refers to a product in which a powder composition obtained as described above is dispersed in an oil agent or, alternately, a product in which a co-modified organopolysiloxane is dissolved or dispersed in an oil agent, and then the powder is added by being mixed and dispersed therein; and a form thereof is that of a liquid dispersed product. This liquid dispersed product is also called a "slurry". In particular, the co-modified organopolysiloxane according to the present invention is useful in that a low-viscosity slurry can be prepared under the same conditions as those of the co-modified organopolysiloxane disclosed in the aforementioned Patent Document 5, even if it is an inorganic powder such as zinc oxide that cannot be sufficiently treated.

The oil agent is not particularly limited provided that a liquid dispersion can be prepared, and is an oil agent that is commonly used as a component of a cosmetic composition. Furthermore, while the oil agent is typically liquid at room temperature, it may be solid such as a wax, and may also be in a highly viscous (high viscosity) gum-like state or paste-like state. The oil agent is preferably one or more selected from (C) silicone oils, nonpolar organic compounds, and low polarity organic compounds that are liquid at 5 to 100°C. In particular, the use of an oil agent containing one or more selected from the following is preferred: (C1) silicone oils that are liquid at 5 to 100°C and (C2) hydrocarbon oils and fatty acid ester oils.

The powder-in-oil dispersion of the present invention can be appropriately prepared according to a known method such as the methods described below.

1. Method of adding and dispersing a powder composition obtained as described above in an ester oil, silicone oil, or other oil agent.

2. Method of dissolving or dispersing the co-modified organopolysiloxane in the oil agent described above, adding the powder thereto, and then mixing the mixture using a ball mill, a bead mill, a sand mill, or other dispersing equipment. The obtained powder-in-oil dispersion can be compounded as-is in a preparation for external use (particularly in a cosmetic composition).

The powder composition and the powder-in-oil dispersion containing the co-modified organopolysiloxane according to the present invention can be suitably used as a topical agent composition, and particularly as a cosmetic material or a cosmetic raw material.

(B) Powder or coloring agent

The powder or coloring agent (B) used in the powder composition, the powder-in-oil dispersion, and the like according to the present invention is a component that is commonly used in a cosmetic composition and includes white and colored pigments as well as extender pigments. The white and colored pigments are used for coloring cosmetic compositions and the like, while extender pigments are used for improving the tactile sensation of the cosmetic compositions. As the "powder" of the present invention, white and colored pigments commonly used in cosmetic compositions, as well as extender pigments can be used without particular limitation. In the present invention, preferably, one or two or more of the powders are compounded. The form (sphere, bar, needle, plate, amorphous, spindle, cocoon, or the like), particle size (aerosol, micro-particle, pigment-grade particle, or the like), and particle structure (porous, nonporous, or the like) of the powder are not limited in any way, but an average primary particle size is preferably in a range of 1 nm to 100 μm. Particularly, when compounding the powder or coloring agent as a pigment, preferably one or two or more

selected from an inorganic pigment powder, an organic pigment powder, and a resin powder having an average particle size in a range of 1 nm to 20 μm is compounded.

Examples of the powder include inorganic powders, organic powders, surfactant metal salt powders (metallic soaps), colored pigments, pearl pigments, metal powder pigments, and the like. Compounded products of these pigments can be used. Furthermore, the surfaces of these pigments may be water-repellent treated.

Specific examples thereof include the same powders or coloring agents disclosed in paragraphs [0150] to [0152] of Patent Document 5 (International Published Patent WO2011/049248) above by the present applicants. The powder used in the present invention is preferably an inorganic powder having ultraviolet absorption/scattering capability on titanium oxide, zinc oxide, iron oxide, cerium oxide, and composites thereof, and particularly preferably those subjected to water repellency surface treatment. The composite refers to a surface treatment powder, a powder in which other inorganic particles are dispersed within an inorganic powder, and the like.

The mixture of the co-modified organopolysiloxane (A) and the powder or coloring agent (B) is a form in which the powder is dispersed in the co-modified organopolysiloxane, and a compounded amount of the powder in the mixture is not particularly limited but is preferably in a range from 50 to 99 wt.% and more preferably in a range from 80 to 90 wt.% of the entire mixture.

(C) Oil agent

The oil agent used in the powder-in-oil dispersion and the like according to the present invention is preferably one or more oil agent selected from silicone oils, nonpolar organic compounds, and low polarity organic compounds that are liquid at 5 to 100°C. A hydrocarbon oil and fatty acid ester oil are preferable as the nonpolar organic compound and the low polarity organic compound.

In particular, the co-modified organopolysiloxane according to the present invention has excellent affinity for oil agents containing one or more selected from not only (C1) a silicone oil which is a liquid at 5 to 100°C but also (C2) hydrocarbon oil and fatty acid ester oil, and can form a stable powder-in-oil dispersion (slurry and the like). Therefore, the co-modified organopolysiloxane is extremely useful as a mixed oil agent of the oil agent serving as component (C1) and oil agent serving as component (C2), or as a cosmetic raw material or topical agent composition containing these.

It is possible to use the oil agent in combination with one or more types of compounds selected from known vegetable oils and fats, animal oils and fats, higher alcohols, liquid fatty acid triglycerides, artificial sebum, and fluorine-based oils. The co-modified organopolysiloxane also displays superior dispersibility in these non-silicone-based oil agents and, therefore the hydrocarbon oil and the fatty acid ester oil can be stably compounded in a cosmetic composition and moisturizing characteristics imparted by these non-silicone-based oil agents can be maintained. Thus, the co-modified organopolysiloxane can improve stability over time of these non-silicone-based oil agents in a cosmetic composition.

By combining the hydrocarbon oil and/or the fatty acid ester oil with the silicone oil, in addition to the dry tactile sensation unique to silicone oils, moisture will be retained on the skin and a moisturizing feel whereby the skin or hair feels moisturized (also referred to as a luxurious tactile sensation) and smooth tactile sensation can be imparted to the cosmetic composition of the present invention. Moreover, there is a benefit in that stability over time of the cosmetic composition will not be negatively affected. Furthermore, with a cosmetic composition comprising the hydrocarbon oil and/or the fatty acid ester oil and the silicone oil, these moisturizing components (the hydrocarbon oil and/or the fatty acid ester oil) can be applied on the skin or hair in a more stable and uniform manner. Therefore, the moisturizing effects of the moisturizing components on the skin are improved. Thus, compared to a cosmetic composition comprising only a non silicone-based oil agent (e.g. a hydrocarbon oil, a fatty acid ester oil, or the like), the cosmetic composition comprising a non silicone-based oil agent along with a silicone oil is advantageous in that a smoother, more luxurious tactile sensation is imparted.

These oil agents are common to those disclosed in paragraphs [0130] to [0135] and paragraph [0206] and the like of Patent Document 5 (International Published Patent WO/2011/049248) by the applicants. Examples of the fluorine-based oil include perfluoropolyether, perfluorodecalin, perfluorooctane, and the like.

A compounded amount of the oil agent in the powder-in-oil dispersion of the present invention is not particularly limited but is preferably in a range from 0.1 to 50 wt.% and more preferably in a range from 0.5 to 25 wt.% in the raw material for use in cosmetic compositions.

The co-modified organopolysiloxane and the powder composition or the powder-in-oil dispersion comprising the co-modified organopolysiloxane can be suitably used as a preparation for external use, particularly for a cosmetic composition or a cosmetic raw material. Such preparations for external use, particularly cosmetic compositions or medicaments are within the scope of the present invention.

In particular, the co-modified organopolysiloxane and powder composition or powder-in-oil dispersion containing the co-modified organopolysiloxane can be suitably used as a make-up cosmetic raw material. The topical agent composition (including cosmetic materials and medicaments), and particularly various make-up cosmetic raw materials

containing these are particularly within the scope of the preferred embodiments of the present invention.

The cosmetic material of the present invention can further contain (D) water, ethanol or other alcohol (including use as a premix), (E) another surfactant, (F) a polyhydric alcohol and/or lower monohydric alcohol, (G) inorganic salts and/or organic acid salt, (H) a silicone component other than component (A), such as cross-linkable organopolysiloxanes, organopolysiloxane elastomer spherical powders, silicone resin, acrylic silicone dendrimer copolymers, silicone raw rubbers, polyamide-modified silicone, alkyl-modified silicone waxes, alkyl-modified silicone resin waves, silicone polyether elastomer gels, PITUITOUS SILICONE FLUIDS, liquid and micro-cross-linkable organopolysiloxanes, and the like, (J) water-soluble polymers, and (K) ultraviolet light blocking components (including combinations of an inorganic and organic ultraviolet light blocking component / ultraviolet light blocking component corresponding to UV-A with a ultraviolet light blocking component corresponding to UV-B), which are common with those disclosed in paragraphs [0101] to [0113] of Patent Document 7 (Japanese Unexamined Patent Application 2013-151657) described above.

Various components other than the components described above can be used in the cosmetic material of the present invention, provided that such use does not impair the effects of the present invention, with examples thereof including oil-soluble gelling agents, organo-modified clay minerals, preservatives, bioactive components, skin beautifying components, pH adjusting agents, antioxidants, solvents, chelating agents, moisturizing components, perfumes, deodorants, and the like. These optional components for cosmetic products are common to those disclosed by the applicants in the aforementioned Patent Document 7 (Japanese Unexamined Patent Application 2013-151657).

The external use preparation according to the present invention is not particularly limited, provided that it is a composition for application to the human body as a cosmetic composition or a medicament. Specific examples of cosmetic material of the present invention include skin cleansing agent products, skin care products, make-up products, antiperspirant products, ultraviolet light blocking products, and similar skin use cosmetic products; hair use cleansing agent products, hair dressing products, hair use coloration products, hair growth products, hair rinsing products, hair conditioning products, hair treatment products, and similar hair use cosmetic products; and bath use cosmetic products. Examples of the medicament of the present invention include hair regrowth agents, hair growth promoters, analgesics, germicides, anti-inflammatory agents, refreshing agents, and skin anti-aging agents, but are not limited thereto.

The types, forms, and containers of the topical agent composition according to the present invention are common to those disclosed in paragraphs [0230] to [0233] and the like of Patent Document 5 (International Published Patent WO/2011/049248) by the present applicants. However, the co-modified organopolysiloxane is particularly useful as a raw material for various make-up cosmetic materials. Furthermore, the cosmetic material according to the present invention is most preferable as a make-up cosmetic material containing (A) the co-modified organopolysiloxane, (B) a powder or coloring agent, and an oil agent containing at least one selected from (C1) silicone oils, which are liquid at 5 to 100°C, and (C2) hydrocarbon oils and fatty acid ester oils.

## EMBODIMENTS

Hereinafter, the present invention is described in detail with reference to Working Examples and Comparative Examples, but it should be understood that the present invention is not limited to these Working Examples. The viscosity (dynamic viscosity) values are measured at 25°C. Note that in the following compositional formulae, Me3SiO groups (or Me3Si groups) are notated as "M", Me2SiO groups are notated as "D", and MeHSiO groups are notated as "$D^H$". Units in which a methyl group in D is modified by an arbitrary substituent (R) are notated as $D^R$.

(Example 1)

< Synthesis of co-modified organopolysiloxane compound P1 >

31.43 g of a methylhydrogenpolysiloxane expressed by the average compositional formula $MD_{5.2}D^H{}_{8.0}M$, 32.17 g of tritrimethylsiloxyvinylsilane, 27.11 g of hexadecene, and 9.28 g of diglycerin monoallyl ether were placed in a reactor and then heated to 65°C while stirring under a nitrogen flow. 0.02 g of a platinum catalyst was added, and a reaction was performed for 10 hours. The completion of the reaction was confirmed by an alkali decomposition gas generation method (i.e., the remaining Si-H groups are decomposed using a KOH ethanol/water solution, and the reaction rate is calculated from the volume of the produced hydrogen gas). Thereafter, a filtering treatment was performed to obtain diglycerin co-modified organopolysiloxane having a siloxane dendron structure expressed by the average compositional formula $MD_{5.2}D^{R1}{}_{3.2}D^{R2}{}_{1.3}D^{R3}{}_{3.5}M$.

In the formula, $R^1$, $R^2$, and $R^3$ have the structures indicated below.

$R^1$= $-C_2H_4Si(OSiMe_3)_3$
$R^2$ = Hydrophilic group expressed by $-C_3H_6O$--X, where X is a diglycerin portion
$R^3$= $-C_{16}H_{33}$

This product was a light brown viscous semi-transparent liquid.

(Example 2)

< Synthesis of co-modified organopolysiloxane compound P2 >

31.62 g of a methylhydrogenpolysiloxane expressed by the average compositional formula $MD_{5.2}D^{H}_{8.0}M$, 30.34 g of tritrimethylsiloxyvinylsilane, 27.27 g of hexadecene, and 10.77 g of diglycerin monoallyl ether were placed in a reactor and then heated to 65°C while stirring under a nitrogen flow. 0.02 g of a platinum catalyst was added, and a reaction was performed for 10 hours. The completion of the reaction was confirmed by an alkali decomposition gas generation method (i.e., the remaining Si-H groups are decomposed using a KOH ethanol/water solution, and the reaction rate is calculated from the volume of the produced hydrogen gas). Thereafter, a filtering treatment was performed to obtain diglycerin co-modified organopolysiloxane having a siloxane dendron structure expressed by the average compositional formula $MD_{5.2}D^{R1}_{3.0}D_{R2}^{1.5}D_{R3}^{3.5}M$. In the formula, $R^1$, $R^2$, and $R^3$ are the same as in Example 1. This product was a light yellow viscous transparent liquid.

(Example 3)

< Synthesis of co-modified organopolysiloxane compound P 3 >

65.66 g of a methylhydrogenpolysiloxane expressed by the average compositional formula $MD_{18}DH_{3.4}M$, 2.27 g of tritrimethylsiloxyvinylsilane, 25.72 g of hexadecene, and 6.35 g of diglycerin monoallyl ether were placed in a reactor and then heated to 65°C while stirring under a nitrogen flow. 0.02 g of a platinum catalyst was added, and a reaction was performed for 10 hours. The completion of the reaction was confirmed by an alkali decomposition gas generation method (i.e., the remaining Si-H groups are decomposed using a KOH ethanol/water solution, and the reaction rate is calculated from the volume of the produced hydrogen gas). Thereafter, a filtering treatment was performed to obtain diglycerin co-modified organopolysiloxane having a siloxane dendron structure expressed by the average compositional formula $MD_{18}D^{R1}_{0.2}D^{R2}_{0.7}D^{R3}_{2.6}M$. In the formula, $R^1$, $R^2$, and $R^3$ are the same as in Example 1. This product was a light yellow viscous transparent liquid.

(Example 4)

< Synthesis of co-modified organopolysiloxane compound P4 >

57.78 g of a methylhydrogenpolysiloxane expressed by the average compositional formula $MD_{29}D^{H}_{7.3}M$, 11.25 g of tritrimethylsiloxyvinylsilane, 27.43 g of hexadecene, and 3.54 g of diglycerin monoallyl ether were placed in a reactor and then heated to 65°C while stirring under a nitrogen flow. 0.02 g of a platinum catalyst was added, and a reaction was performed for 10 hours. The completion of the reaction was confirmed by an alkali decomposition gas generation method (i.e., the remaining Si-H groups are decomposed using a KOH ethanol/water solution, and the reaction rate is calculated from the volume of the produced hydrogen gas). Thereafter, a filtering treatment was performed to obtain diglycerin co-modified organopolysiloxane having a siloxane dendron structure expressed by the average compositional formula $MD_{29}D^{R1}_{1.6}D^{R2}_{0.7}D^{R3}_{5.0}M$. In the formula, $R^1$, $R^2$, and $R^3$ are the same as in Example 1. This product was a light yellow viscous transparent liquid.

(Comparative Example 1)

< Synthesis of co-modified organopolysiloxane compound R1 >

61.26 g of a methylhydrogenpolysiloxane expressed by the average compositional formula $MD_{15}D^{H}_{3.0}M$, 32.32 g of tritrimethylsiloxyvinylsilane, and 6.42 g of diglycerin monoallyl ether were placed in a reactor and then heated to 65°C while stirring under a nitrogen flow. 0.02 g of a platinum catalyst was added, and a reaction was performed for 10 hours. The completion of the reaction was confirmed by an alkali decomposition gas generation method (i.e., the remaining Si-H groups are decomposed using a KOH ethanol/water solution, and the reaction rate is calculated from the volume of the produced hydrogen gas). Thereafter, a filtering treatment was performed to obtain diglycerin co-modified organopolysiloxane having a siloxane dendron structure expressed by the average compositional formula $MD_{16}D^{R1}_{2.4}D^{R2}_{0.6}M$. In the formula, $R^1$ and $R^2$ are the same as in Example 1. This product was a light yellow viscous transparent liquid.

(Comparative Example 2)

< Synthesis of co-modified organopolysiloxane compound R2 >

58.53 g of a methylhydrogenpolysiloxane expressed by the average compositional formula $MD_{29}D^{H}_{7.3}M$, 7.30 g of tritrimethylsiloxyvinylsilane, 27.72 g of hexadecene, and 6.46 g of diglycerin monoallyl ether were placed in a reactor and then heated to 65°C while stirring under a nitrogen flow. 0.02 g of a platinum catalyst was added, and a reaction was performed for 10 hours.

The completion of the reaction was confirmed by an alkali decomposition gas generation method (i.e., the remaining Si-H groups are decomposed using a KOH ethanol/water solution, and the reaction rate is calculated from the volume of the produced hydrogen gas). Thereafter, a filtering treatment was performed to obtain diglycerin co-modified organopolysiloxane having a siloxane dendron structure expressed by the average compositional formula $MD_{29}D^{R1}_{1.0}D^{R21}_{1.3}D^{R3}_{5.0}M$. In the formula, $R^1$, $R^2$, and $R^3$ are the same as in Example 1. This product was a light yellow viscous transparent liquid.

Table 1 summarizes the average compositional formula, HLB and viscosity of the co-modified organopolysiloxane compounds P1 to P4 obtained from the aforementioned Examples 1 to 4 and the co-modified organopolysiloxane compounds R1 and R2 for comparison obtained from Comparative Examples 1 and 2. R1, R2, and R3 are the same as in Example 1.

[Table 1]

| Co-modified organopolysiloxane | Average composition formula | HLB | Viscosity (mPa-s) |
|---|---|---|---|
| P1 | $MD_{5.2}D^{R1}_{3.2}D^{R2}_{1.3}D^{R3}_{3.5}M$ | 1.7 | 950 |
| P2 | $MD_{5.2}D^{R1}_{3.0}D^{R2}_{1.5}D^{R3}_{3.5}M$ | 2.0 | 1430 |
| P3 | $MD_{18}D^{R1}_{0.2}D^{R2}_{0.7}D^{R3}_{2.6}M$ | 1.2 | 310 |
| P4 | $MD_{29}D^{R1}_{1.6}D^{R2}_{0.7}D^{R3}_{5.0}M$ | 0.6 | 760 |
| R1 | $MD_{16}D^{R1}_{2.4}D^{R2}_{0.6}M$ | 1.0 | 620 |
| R2 | $MD_{29}D^{R1}_{1.0}D^{R2}_{1.3}D^{R3}_{5.0}M$ | 1.2 | 2500 |

[Evaluation of dispersion stability]

Each of the aforementioned co-modified organopolysiloxanes (denoted as P1 to R2 in the table) were dissolved or dispersed in an oil agent (ethylhexyl palmitate) by a composition shown in Table 2, and a powder (titanium dioxide with an average particle diameter of 10 × 90 nm (product name STR-100A-LP, manufactured by Sakai Chemical Industry Co., Ltd.)) and zirconia beads (0.8 mm diameter) were added thereto and mixed for 15 hours in a paint shaker (PAINT SHAKER, manufactured by ASADA IRON WORKS.CO., LTD.) to prepare a powder dispersion (slurry). Table 2 shows the results of performing evaluations based on the following evaluation criteria.

O: Slurry viscosity of less than 2500 mPa·s
△: Slurry viscosity of 2500 mPa·s or more
×: Fluid slurry cannot be prepared

[Table 2]

| No. | Component (g) | Embodiments | | | | Comparative Examples | |
|---|---|---|---|---|---|---|---|
| | | P1-T1 | P2-T1 | P3-T1 | P4-T1 | R1-T1 | R2-T1 |
| 1 | Titanium dioxide with an average particle diameter of 10 × 90 nm (product name STR-100A-LP, Sakai Chemical Industry Co., Ltd.) | 20 | 20 | 20 | 20 | 20 | 20 |
| 2 | Ethylhexyl palmitate | 16 | 16 | 16 | 16 | 16 | 16 |
| 3 | P1 | 4 | | | | | |

(continued)

| No. | Component (g) | Embodiments | | | | Comparative Examples | |
|---|---|---|---|---|---|---|---|
| | | P1-T1 | P2-T1 | P3-T1 | P4-T1 | R1-T1 | R2-T1 |
| 4 | P2 | | 4 | | | | |
| 5 | P3 | | | 4 | | | |
| 6 | P4 | | | | 4 | | |
| 7 | R1 | | | | | 4 | |
| 8 | R2 | | | | | | 4 |
| 9 | Zirconia beads | 160 | 160 | 160 | 160 | 160 | 160 |
| | Slurry evaluation results (Fatty acid ester oil) | ○ | ○ | ○ | ○ | × | △ |
| | Slurry evaluation results (Decamethyl cyclopentasiloxane) | ○○ | ○○ | ○△ | - | - | - |

As shown in Table 1, the co-modified organopolysiloxanes P1 to P4 according to the present invention could form low-viscosity slurries using ethylhexyl palmitate, which is a typical fatty acid ester oil. However, with comparative compounds R1 and R2, slurries could not be prepared or were highly viscous, and thus sufficient powder dispersion performance could not be achieved for fatty acid ester oils.

For the co-modified organopolysiloxanes P1 to P3 according to the present invention, 16 mass parts of decamethylcyclopentasiloxane was used instead of 16 mass parts of ethylhexyl palmitate, and the evaluation results of prototype slurry production using the same method are also shown in Table 1. The evaluation criteria in this case are as follows.

OO: Slurry viscosity less than 1000 mPa s, no thickening over time.
○△: Slurry viscosity less than 1000 mPa s, thickening over time.

As shown in Table 2, the co-modified organopolysiloxanes according to the present invention, particularly P1 and P2, can form slurries with low viscosity and excellent stability over time, even with decamethylcyclopentasiloxane, and have excellent powder dispersion performance with silicone and non-silicone oil agents. Thus, the dispersion stability of powders is expected to improve in topical agent compositions and cosmetic materials that use or combine these oil agents.

[Formulation Examples]

Other cosmetic materials using the co-modified organopolysiloxane of the present invention are disclosed in Japan Patent Application 2020-160317, including [Formulation Example 1] W/O cushion foundation, [Formulation Example 2] W/O sunscreen agent, and [Other Formulation Examples].

## Claims

1. A co-modified organopolysiloxane expressed by structural formula (1) below, wherein the value of HLB defined as

$$\text{HLB} = (\text{Total molecular weight of hydrophilic group portions} / \text{Total molecular weight}) \times 20$$

is within a range of 0.5 to 2.0, and the viscosity at 25°C is within a range of 200 to 1500 mPa·s.
Structural formula (1):

[Formula 1]

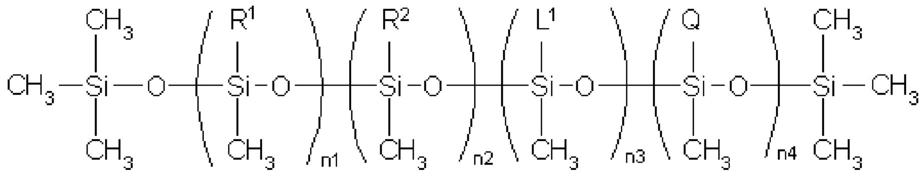

{where

$R^1$ is a substituted or unsubstituted monovalent hydrocarbon group having 1 to 30 carbon atoms, or a hydrogen atom;
$R^2$ represents a substituted or unsubstituted straight-chain or branched monovalent hydrocarbon group having 6 to 30 carbon atoms;
$L^1$ represents one or more type of group selected from:
a silylalkyl group having the siloxane dendron structure expressed by the following general formula (2) when i = 1:

[Formula 2]

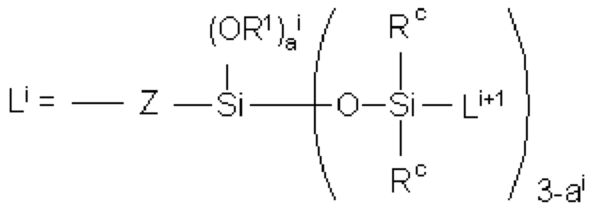

(2)

(where $R^1$ is a group similar to the groups described above, $R^C$ is an alkyl group with 1 to 6 carbon atoms, or a phenyl group, and Z is a divalent organic group. i in L' represents the number of units of silylalkyl groups represented by L' and is an integer from 1 to c, where c represents the number of units, or in other words the number of repetitions of silylalkyl groups, and where the number of units c is an integer from 1 to 10. $L^{i+1}$ represents a silylalkyl group where i is less than c and is a methyl group or a phenyl group when i=c. $a^i$ is a number from 0 to 3);
a chain organosiloxane group, expressed by the following general formula (2'):

[Formula 3]

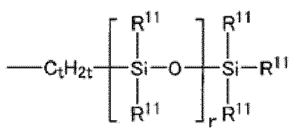

(2')

(where $R^{11}$ are each independently a substituted or unsubstituted monovalent hydrocarbon group having 1 to 30 carbon atoms, hydroxyl group, or hydrogen atom, and at least one of the $R^{11}$ groups is a monovalent

hydrocarbon group; t is a number in a range of 2 to 10; and r is a number in a range of 1 to 500); and
a chain type organosiloxane group expressed by the following general formula (2"):

[Formula 4]

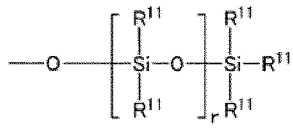

(2")

(where $R^{11}$ and r are similar to those described above);
Q is a hydrophilic group containing a sugar alcohol expressed by the following structural formulae (3-1) to (3-2):

[Formula 5]

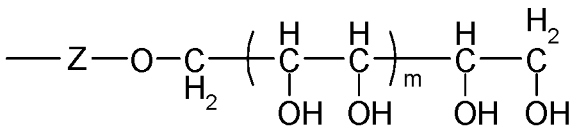

(3-1)

(where Z represents an alkylene group having 2 to 22 carbon atoms or a divalent organic group expressed by $-R^6-C(=O)-O-R^7-$ ($R^6$ is an alkylene group having 2 to 22 carbon atoms, $R^7$ is a group selected from ethylene groups, propylene groups, methylethylene groups, and hexylene groups), and m is 1 or 2)

[Formula 6]

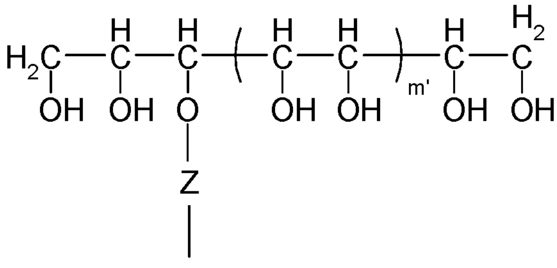

(3-2)

(where Z is similar to those described above, and m' is 0 or 1),
and
a hydrophilic group containing at least one hydrophilic unit selected from hydrophilic units expressed by the following structural formulae (3-3) to (3-5):

[Formula 7]

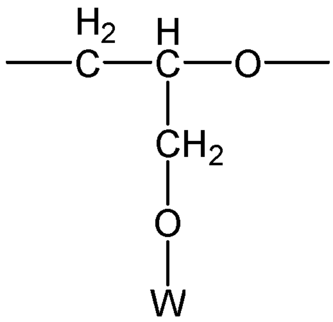

(3-3)

(where W is a hydrogen atom or an alkyl group having 1 to 20 carbon atoms)

[Formula 8]

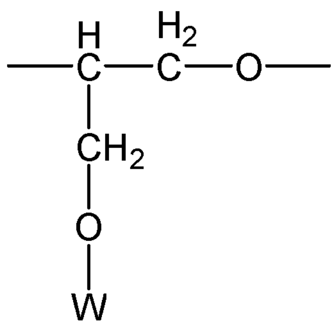

(3-4)

[Formula 9]

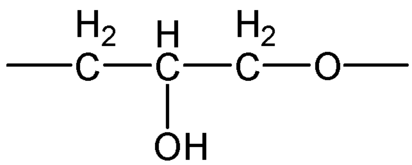

(3-5)

where the hydrophilic groups are being bonded to silicon atom via a divalent linking group (Z); and (n1 + n2 + n3 + n4) is a number with a range of 2 to 50, n1 is a number within a range of 0 to 20, n2 is a number within a range of 1 to 10, n3 is a number within a range of 0.1 to 10, and n4 is a number within a range of 0.3 to 2.5.}

**2.** The co-modified organopolysiloxane according to claim 1, wherein in formula (1), $L^1$ is a functional group expressed by the following general formula (2-1) or general formula (2-2).
General Formula (2-1):

[Formula 10]

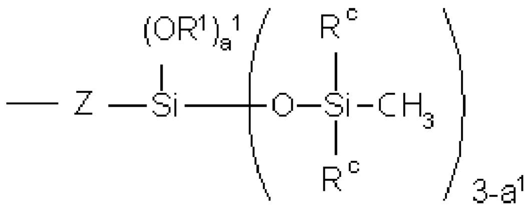

(2-1)

General Formula (2-2):

[Formula 11]

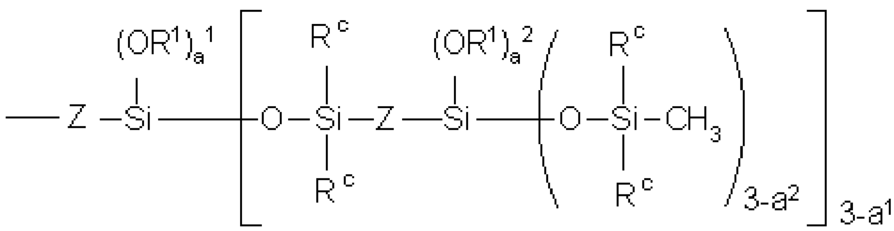

(2-2)

(where $R^1$, $R^C$, and Z are similar to the groups described above, and a' and $a^2$ are both independently a number within a range of 0 to 3.)

**3.** The co-modified organopolysiloxane according to claim 1 or 2, wherein in the aforementioned general formula (1), Q is a diglycerin derivative group-containing organic group (excluding those having an oxyalkylene structure having an average number of repetitions of an oxyalkylene unit of 2 or higher) containing at least one glycerin unit selected from glycerin units expressed by the aforementioned structural formulae (4-1) to (4-3) with the number of repetitions thereof being in a range of on average within 1.5 to 2.4 (excluding those having an oxyalkylene structure having an average number of repetitions of an oxyalkylene unit of 2 or higher), which is bonded to a silicon atom via a linking group that is at least divalent (excluding those having an oxyalkylene structure with two or more repetitions of the oxyalkylene units).

**4.** The co-modified organopolysiloxane according to any one of claims 1 to 3, wherein in the aforementioned general formula (1), Q is a diglycerin derivative group-containing organic group expressed by the following general formula (5-1)

[Formula 12]

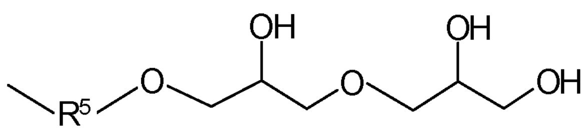

(5-1)

(where $R^5$ represents a divalent organic group (excluding those having an oxyalkylene structure with two or more repetitions of the oxyalkylene units),
or
the following general formula (5-2)

[Formula 13]

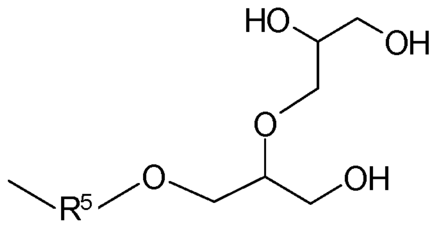

(5-2)

(where $R^5$ is similar to those described above).

**5.** The co-modified organopolysiloxane according to any one of claims 1 to 4, wherein the HLB value is within a range of 1.5 to 2.0, $R^2$ in general formula (1) is an alkyl group having 8 to 20 carbon atoms, and the viscosity at 25°C is within a range of 700 to 1450 mPa s.

**6.** A powder treatment agent, comprising the co-modified organopolysiloxane according to any one of claims 1 to 5.

**7.** A surfactant or surface treatment agent, comprising the co-modified organopolysiloxane according to any one of claims 1 to 5.

**8.** A powder composition, comprising:

(A) the co-modified organopolysiloxane according to any one of claims 1 to 5; and
(B) a powder or a coloring agent.

**9.** A powder-in-oil dispersion, comprising:

(A) the co-modified organopolysiloxane according to any one of claims 1 to 5;
(B) a powder or coloring agent; and
(C) one or more oil agents that are a liquid at 5 to 100°C, selected from silicone oils, nonpolar organic compounds, and low polarity organic compounds.

**10.** A topical agent composition, comprising the co-modified organopolysiloxane according to any one of claims 1 to 5.

**11.** A topical agent composition according to claim 10, comprising:

(A) the co-modified organopolysiloxane according to any one of claims 1 to 5;
(B) a powder or coloring agent; and
(C) one or more oil agents that are a liquid at 5 to 100°C, selected from silicone oils, nonpolar organic compounds, and low polarity organic compounds.

## INTERNATIONAL SEARCH REPORT

International application No.
**PCT/JP2021/037003**

**A. CLASSIFICATION OF SUBJECT MATTER**

***C08G 77/38***(2006.01)i; ***C08G 77/50***(2006.01)i; ***C08L 83/10***(2006.01)i; ***C08L 83/14***(2006.01)i; ***A61K 8/892***(2006.01)i; ***C08K 3/00***(2018.01)i
FI: C08G77/50; C08G77/38; C08L83/14; C08L83/10; C08K3/00; A61K8/892

According to International Patent Classification (IPC) or to both national classification and IPC

**B. FIELDS SEARCHED**

Minimum documentation searched (classification system followed by classification symbols)

C08G77/38; C08G77/50; C08L83/10; C08L83/14; A61K8/892; C08K3/00

Documentation searched other than minimum documentation to the extent that such documents are included in the fields searched

Published examined utility model applications of Japan 1922-1996
Published unexamined utility model applications of Japan 1971-2021
Registered utility model specifications of Japan 1996-2021
Published registered utility model applications of Japan 1994-2021

Electronic data base consulted during the international search (name of data base and, where practicable, search terms used)

**C. DOCUMENTS CONSIDERED TO BE RELEVANT**

| Category* | Citation of document, with indication, where appropriate, of the relevant passages | Relevant to claim No. |
|---|---|---|
| X | JP 2013-151657 A (DOW CORNING TORAY CO LTD) 08 August 2013 (2013-08-08) claims 1-11, 14-16, examples 6, 7, table 1 | 1-11 |
| A | JP 2012-246445 A (DOW CORNING TORAY CO LTD) 13 December 2012 (2012-12-13) claims, examples | 1-11 |
| A | JP 2012-46508 A (DOW CORNING TORAY CO LTD) 08 March 2012 (2012-03-08) claims, examples | 1-11 |
| A | JP 2011-246706 A (DOW CORNING TORAY CO LTD) 08 December 2011 (2011-12-08) claims, examples | 1-11 |

☐ Further documents are listed in the continuation of Box C.  ☑ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **18 November 2021** | **07 December 2021** |

| Name and mailing address of the ISA/JP | Authorized officer |
|---|---|
| **Japan Patent Office (ISA/JP)**<br>**3-4-3 Kasumigaseki, Chiyoda-ku, Tokyo 100-8915**<br>**Japan** | Telephone No. |

Form PCT/ISA/210 (second sheet) (January 2015)

**INTERNATIONAL SEARCH REPORT**
**Information on patent family members**

International application No.
**PCT/JP2021/037003**

| Patent document cited in search report | Publication date (day/month/year) | Patent family member(s) | Publication date (day/month/year) |
|---|---|---|---|
| JP 2013-151657 A | 08 August 2013 | WO 2013/100177 A1<br>claims 1-11, 14-16, examples 6, 7, table 1<br>US 2014/0371330 A1<br>CN 104136499 A<br>KR 10-2014-0116889 A | |
| JP 2012-246445 A | 13 December 2012 | WO 2012/165227 A1 | |
| JP 2012-46508 A | 08 March 2012 | WO 2012/015069 A1<br>claims, examples<br>US 2013/0177516 A1<br>CN 103079539 A<br>KR 10-2013-0099013 A | |
| JP 2011-246706 A | 08 December 2011 | WO 2011/136393 A1<br>claims, examples<br>US 2013/0210930 A1<br>CN 102947369 A<br>KR 10-2013-0058702 A | |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- JP H0753326 A
- JP 2719303 B
- JP H10167946 A
- JP 2002038013 A
- WO 2011049246 A
- WO 2011049248 A
- WO 2011136394 A
- JP 2013151657 A
- JP 6369887 B
- JP 2020160317 A